# FASCICULE DE BREVET EUROPEEN

(11) **EP 0 648 273 B1**
(45) Date de publication et mention de la délivrance du brevet: **28.08.1996**
(21) Numéro de dépôt: 93913124.9
(22) Date de dépôt: 14.06.1993
(51) Int. Cl.: C12P 7/18

(54) **Procédé pour l'obtention de produits à activité bactérienne capables de transformer le glycérol en 1,3-propanediol, souches correspondantes et application à la production industrielle d'1,3-propanediol**
Verfahren zur Herstellung von Erzeugnissen mit bakterieller Aktivität, die zur Umwandlung von Glycerin in 1,3-Propandiol fähig sind, sowie die entsprechenden Stämme und deren Anwendung zur industrieller Herstellung von 1,3-Propandiol
Process for the production of products having bacterial activity and capable of transforming glycerol into 1,3-propanediol, corresponding strains, and application in the industrial production of 1,3-propanediol

(30) Priorité: 15.06.1992 FR 9207212
(43) Date de publication de la demande: 19.04.1995
(73) Titulaire: INSTITUT NATIONAL DE LA RECHERCHE AGRONOMIQUE, F-75341 Paris Cédex 07 (FR)
(72) Inventeur: BORIES, André, F-11110 Armissan (FR); CLARET, Carole, F-11110 Vinassan (FR)
(74) Mandataire: Phélip, Bruno
(86) Numéro de dépôt international: FR9300568
(87) Numéro de publication internationale: WO9325696

(56) Documents cités:
- EP-A- 0 361 082
- APPLIED MICROBIOLOGY AND BIOTECHNOLOGY. vol. 36, no. 5, Février 1992, SPRINGER INTERNATIONAL. pages 592 - 597 H. BIEBL ET AL. 'Glycerol conversion to 1,3-propanediol by newly isolated clostridia.'
- APPLIED MICROBIOLOGY AND BIOTECHNOLOGY. vol. 36, no. 3, Décembre 1991, SPRINGER INTERNATIONAL. pages 289 - 294 B.G]NZEL ET AL. 'Fermentative production of 1,3-propanediol from glycerol by Clostridium butyricum up to a scale of 2m3.'

## Description

La présente invention concerne la production de 1,3- propanediol par fermentation de glycérol . Elle a plus particulièrement pour objet l'obtention de produits à activité bactérienne, capables de transformer le glycérol en 1,3- propanediol et leur application à la production industrielle de 1,3-propanediol . Les produits à activité bactérienne obtenus selon l'invention sont des populations microbiennes représentées par des écosystèmes anaérobies de digesteurs de résidus agro-industriels, de sédiments et de boues, ou de nouvelles souches pures issues de ces milieux . Parmi ces dernières , on peut notamment citer : **Enterobacter agglomerans**, biogroupe V et **Clostridium butyricum**.

L'invention a encore pour objet la production industrielle de 1,3-propanediol à partir de glycérol de co-produits et résidus de transformation de matières d'origine végétale ou animale, et en particulier les co-produits de distillation d'alcool d'origine agricole (éthanol industriel ou bioéthanol, alcools de bouche et eaux de vie , et substances analogues).

Le 1,3-propanediol ou triméthylèneglycol est un produit de base pour la synthèse des polyuréthanes et des polyesters , qui se substitue avantageusement aux matières premières actuellement utilisées, comme l'éthylèneglycol, le 1,2-propanediol et le 1,4-butanediol, grâce aux caractéristiques qu'il confère aux produits obtenus: résistance des fibres , élasticité, résistance à la lumière, à la corrosion, ... Il s'utilise également comme additif dans la préparation de produits agro-alimentaires, et pharmaceutiques ( agent humectant par exemple ) dans les aliments pour animaux, le tabac, les préparations pharmaceutiques ....

La synthèse chimique de 1,3-propanediol s'effectue à partir de l'acroléine, matière très toxique , et issue de sources fossiles . Elle nécessite plusieurs étapes : déshydratation en hydroxypropanal puis hydrogénation catalytique, mettant en oeuvre des réactions délicates à réaliser et dont les principaux inconvénients sont les risques dûs à l'acroléine et aux produits secondaires, les conditions strictes de synthèse , la contamination éventuelle du produit fini par des substances toxiques, ce qui réduit les usages du 1,3-propanediol.

La formation de 1,3-propanediol a été mise en évidence ces dernières années chez quelques bactéries anaérobies facultatives et strictes, se développant à partir de glycérol comme substrat carboné . Mais le nombre de bactéries produisant du 1,3-propanediol et les connaissances sur les conditions de production sont encore restreints. En effet, seulement trois genres principaux sont connus : **Clostridium**, **Klebsiella**, **Citrobacter**, choix réduit qui limite les possibilités d'amélioration de la production.

Les premiers travaux relatant la formation microbienne de 1,3-propanediol ont été présentés par Magazanik et col., 1954 , (J. Bacteriol., 66, 611-619), chez Klebsiella pneumoniae , bactérie anaérobie facultative . La voie métabolique de biosynthèse du 1,3-propanediol a été ultérieurement caractérisée par Forage et Foster, 1982, ( J. Bacteriol., 149, 413-419 ) , et elle comprend deux étapes enzymatiques effectuées par une glycérol-déshydratase à coenzyme B12 dépendante et une 1,3-propanediol oxydoréductase à NAD . Le rendement de conversion du glycérol comme monosubstrat en 1,3- propanediol est chez cette bactérie d'environ 40-45 % ( poids/ poids) . Les autres produits formés par la bactérie à partir du glycérol sont : l'acétate, le lactate, l'éthanol, le 2,3- butanediol.

Dans le genre **Citrobacter**, une espèce a été décrite comme formant du 1,3-propanediol ; **Citrobacter freundii** (Homann et col., 1990, Appl. Microbiol. Biotechnol., 33, 121-126). Le rendement de conversion à partir de glycérol comme monosubstrat est proche de 50% ( poids/poids) , avec formation prépondérante d'acétate comme principal co-produit fermentaire, ainsi que des produits secondaires comme l'éthanol et le lactate . A la connaissance du demandeur , les seuls micro-organismes anaérobies stricts cités dans la littérature fermentant le glycérol en 1,3-propanediol appartiennent au genre Clostridium et les espèces sont : C. acetobutylicum, C. beijerinckii, C. butylicum, C. butyricum, C. kantontoi.

Comme document illustrant l'état de la technique dans le domaine de l'invention, on peut également citer la demande de brevet EP-A-O 361.082 qui décrit un procédé de sélection de souches bactériennes disponibles, en particulier dans des collections de cultures, pour leur aptitude à transformer le glycérol en 1,3-propanediol. Selon ce procédé, on réalise une fermentation dans des conditions habituelles, en présence d'une des souches à sélectionner, d'une solution de glycérine à 5% comme seule source de carbone. On sélectionne les souches qui procurent le meilleur rendement de transformation en 1,3- propanediol.

On connaît aussi un procédé de sélection de souches à partir d'échantillons de paille en décomposition et de compost . Néanmoins ce procédé décrit par Biebl et al. (1991, Appl. Microb. Biol., 36, n°5, 592-597) présente l'inconvénient notable de nécessiter une pasteurisation des échantillons avant leur mise en culture . Seuls les microorganismes sporulés survivent à cette opération tandis que de nombreux genres , tels que Citrobacter et Klebsiella, sont détruits .

Ce procédé élimine ainsi avant l'étape de sélection des micro-organismes pouvant présenter des caractéristiques intéressantes . Il est donc très spécifique et ne permet la sélection que dans une gamme restreinte de micro-organismes.

On notera de plus que ce document ne mentionne l'isolement de souches qu'à partir d'habitats , tels que de la paille en décomposition ou du compost, où les organismes sont en présence d'air et non à partir d'habitats anaérobies.

A partir de glycérol comme seul substrat carboné, la voie fermentaire chez Clostridium conduit respectivement à :
- la synthèse de 1,3-propanediol , voie assurant la régénération des coenzymes réduits (NADH),
- la formation de métabolites : butyrate, acétate, éthanol, butanol, acétone, dioxyde de carbone et hydrogène , en proportion variable selon les espèces et les conditions, mais qui résultent directement ou indirectement des voies obligées de production d'énergie (ATP) générant également les coenzymes réduits . Les tentatives d'apport de co-substrats carbonés, tels les oses, pour favoriser les voies de production d'énergie et orienter préférentiellement la synthèse du 1,3-propanediol à partir de glycérol, conduisent à l'accumulation de ces produits secondaires, facteurs potentiels d'inhibition des bactéries ( Biebl M., 1991, Appl. Microb. Biol., 35, 701-705).

Les conditions de mise en oeuvre de ces souches bactériennes, en cultures pures anaérobies, constituent des handicaps à l'échelle de la production de 1,3-propanediol en condition industrielle . Il est en effet indispensable d'opérer dans des conditions de stérilité pour éviter les contaminations, d'acclimater les souches aux milieux de production et d'adapter les compositions et paramètres des milieux . En outre , les souches bactériennes sont sensibles aux inhibiteurs du milieu fermentaire, initialement présents ou formés.

L'invention concerne l'obtention de bactéries anaérobies facultatives et strictes fermentant le glycérol et la production de 1,3-propanediol par des espèces et des souches nouvellement décrites et obtenues, en cultures pures ou mixtes . Ce procédé est destiné à la production de 1,3- propanediol principalement à partir du glycérol contenu dans des substances issues de procédés industriels de transformation de matières d'origines végétales , ou de milieux à base de glycérol purifié.

Jusqu'à présent , à la connaissance du demandeur , aucun procédé de production de 1,3 propanediol n'a fait référence à des méthodes d'obtention, d'isolement et d'utilisation des souches microbiennes à partir d'habitats microbiens anaérobies. Le nombre restreint de souches de collection productrices de 1,3 propanediol entraîne des difficultés d'acclimatation des souches à des milieux aussi variés que ceux d'origine industrielle.

Sous un premier aspect, la présente invention a pour objet un procédé pour l'obtention de produits à activité bactérienne, capables de transformer le glycérol en 1,3- propanediol, ledit procédé comprenant les étapes de (a) préculture de populations anaérobies, issues d'habitats microbiens anaérobies, ladite préculture étant réalisée dans des conditions anaérobies sur milieu nutritif tamponné , contenant du glycérol comme seule source de carbone , (b) isolement des précultures microbiennes actives capables de fermenter le glycérol (c) enrichissement par fermentation discontinue desdites précultures dans un réacteur anaérobie , sur milieu nutritif à base de glycérol comme substrat et à pH régulé (d) isolement des produits à activité bactérienne capables de transformer le glycérol en 1,3-propanediol.

L'invention part de l'observation selon laquelle la fermentation du glycérol en 1,3-propanediol est une étape préalable principale de la dégradation qui prend place dans des écosystèmes anaérobies.

La présente invention consiste à obtenir des produits à activité bactérienne , et en particulier des souches pures bactériennes, fermentant le glycérol à partir d'habitats microbiens anaérobies représentés par exemple par des digesteurs anaérobies, des sédiments de milieux naturels (sols, et autres ) et de boues de bassin de stockage d'effluents .

Selon l'invention, des précultures de populations anaérobies issues d'habitats microbiens anaérobies sont réalisées en conditions anaérobies sur milieu nutritif tamponné, à base de glycérol comme seule source de carbone et d'énergie, dans le but de favoriser le développement des souches capables de fermenter le glycérol. A partir des précultures actives , une phase d'enrichissement est effectuée en fermentation discontinue ( batch ) en réacteur anaérobie, sur milieu nutritif à base de glycérol comme substrat , et à pH régulé.

Les conditions pratiques de pH varient selon la nature des microorganismes isolés . Des pH compris entre 5 et 8, et de préférence entre 6 et 7,5 se sont avérés les plus appropriés .

Durant la phase de consommation du glycérol par la culture enrichie en microorganismes fermentant le glycérol, l'isolement de souches bactériennes est opéré à partir de prélèvements en anaérobiose, et mettant en oeuvre une méthode de dilution des 5 populations puis un étalement sur boîtes de Pétri selon une technique de simple ou double couche de milieu de culture gélosé , permettant de respecter les conditions anaérobies adaptées aux germes anaérobies facultatifs ou stricts.

La présente invention concerne les produits et bactéries ainsi obtenus, à partir de sources microbiennes anaérobies capables de produire du 1,3 propanediol et qui appartiennent en particulier aux espèces suivantes : Enterobacter agglomerans, Clostridium butyricum, Citrobacter amalonaticus.

Sous un autre aspect, l'invention concerne également de nouvelles souches bactériennes identifiées par leur numéro de dépôt auprès de la Collection Nationale de Cultures de Microorganismes de l'Institut Pasteur ( CNCM), 25, rue du Docteur Roux ,PARIS, à savoir :
- la souche **Enterobacter agglomerans**, biogroupe V (souche PPDAF- INRA ) , déposée sous le N I-1210 le 20 Mai 1992,
- la souche Clostridum butyricum ( souche PPDAS- INRA), déposée sous le n° I-1211 le 20 Mai 1992,
- la souche Citrobacter amalonaticus ( souche GAF-INRA) déposée sous le n° I-1212 le 20 Mai 1992.

Sous encore un autre aspect de l'invention , le procédé de production de 1,3-propanediol comprend la mise en oeuvre des produits à activité bactérienne, que ce soit en cultures pures - en particulier des bactéries Enterobacter agglomerans, Clostridium butyricum - , ou en cultures mixtes d'écosystèmes anaérobies qui contiennent au moins une des espèces ou souches des bactéries : Enterobacter agglomerans , Clostridium butyricum, Citrobacter amalonaticus.

Le procédé selon l'invention peut utiliser l'espèce bactérienne Enterobacter agglomerans et plus particulièrement la nouvelle souche Enterobacter agglomerans pour la production de 1,3-propanediol à partir de glycérol comme seule source de carbone assimilé, espèce nouvellement décrite pour sa capacité à fermenter le glycérol en 1,3-propanediol. La mise en oeuvre des souches Enterobacter agglomerans est réalisée en conditions d'anaérobiose sur milieu de culture contenant du cobalt et en particulier sur milieu minéral additionné de 0,45 mg/l de cobalt et avec contrôle et régulation du pH entre 6 et 7,5 et de préférence à pH 7, dans une gamme de température située entre 28 et 40°C environ.

La nouvelle souche Enterobacter agglomerans se caractérise par un rendement de production de 1,3-propanediol d'au moins 74 mM pour 100 mM de glycérol consommé et une productivité volumique d'au moins 2 g de propanediol par litre et par heure en phase active de fermentation ( glycérol à 2%). L'analyse du profil fermentaire révèle que la biosynthèse de 1,3-propanediol s'accompagne de la formation exclusive d'acide acétique comme co-métabolite majeur dosé dans la phase aqueuse, ce qui constitue un moyen de simplifier la phase de purification . La souche montre de plus un caractère gazogène peu élevé à partir de glycérol.

L'ensemble des caractéristiques relatives aux conditions et performances fermentaires du glycérol en 1,3-propanediol décrites ci-dessus : rendement très élevé, forte productivité, acétate comme seul co-produit accumulé , confèrent au procédé selon l'invention mettant en oeuvre l'espèce Enterobacter agglomerans et plus particulièrement la nouvelle souche Enterobacter agglomerans , des meilleures possibilités d'utilisation pour la production de propanediol à partir de glycérol comme source de carbone assimilable, en vue de l'application industrielle à des solutions de glycérol purifié ou contenu dans les eaux de procédés de transformation de matières organiques renouvelables.

Dans le procédé selon l'invention on peut également utiliser la nouvelle souche Clostridium butyricum, pour la production de propanediol à partir de glycérol présent dans des sous-produits d'origine agro-industrielle ou à partir de glycérol purifié, selon les conditions de fermentation suivantes :
- anaérobiose stricte - purge du milieu par flux d'azote, ajout de composés rédacteurs ,
- pH régulé à des valeurs comprises entre 5 et 8 et de préférence entre 6 et 7,5,
- température : située entre 32 et 40°C, environ, notamment voisine de 37°C.

Le milieu de fermentation contient des éléments minéraux cofacteurs de l'extrait de levure et du glycérol à des concentrations comprises entre 15 et 200 g/l.

Il peut contenir en outre d'autres substances organiques soit non assimilées, soit capables d'être utilisées en tant que co-substrat.

Les rendements de conversion du glycérol en 1,3-propanediol obtenus sur milieu d'origine industrielle sont semblables à ceux déterminés en milieu synthétique : 64 % et 57% respectivement ( rendement molaire ).

Dans les conditions conformes à l'invention, la production de propanediol à partir de glycérol comme seul substrat carboné par Clostridium butyricum est réalisée pour une concentration initiale en glycérol allant jusqu'à 200 g/l, de préférence entre 65 et 180 g/l, avec une productivité volumique comprise entre 4,5 et 2,3 g de propanediol par litre de fermenteur et par heure , durant la phase active de fermentation.

Alors que les souches du genre Clostridium fermentant le glycérol en 1,3-propanediol répertoriées dans la bibliographie produisent un grand nombre de sous-produits : acétate, butyrate , éthanol, lactate, ..., le procédé selon l'invention conduit à la formation d'un seul co-métabolite majeur , l'acide butyrique pouvant faciliter les opérations ultérieures de récupération du 1,3-propanediol.

Le procédé faisant l'objet de la présente invention peut mettre en oeuvre toute population microbienne anaérobie d'origine naturelle ou industrielle dans laquelle la présence de l'espèce Enterobacter agglomerans ou de Clostridium butyricum est démontrée.

Ce procédé de production de propanediol à partir des populations mixtes ainsi définies, permet une simplification de la mise en oeuvre des fermentations par diminution du risque de contamination.

La production de 1,3-propanediol est effectuée à pH compris entre 5 et 8, et de préférence entre 6,5 et 7, pour une concentration en glycérol comprise entre 20 et 125 g/l. A 37 °C , et à pH 6,5, le rendement optimal de conversion du glycérol en propanediol est de 66,7 %, lorsque la fermentation est réalisée avec 2% de glycérol. La plus forte productivité obtenue pour une culture à 50 g/l de glycérol est de 2,4 g/l/h.

L'invention sera encore illustrée sans être aucunement limitée par les exemples qui suivent :

### EXEMPLE 1:

### Obtention et isolement de souches bactériennes à partir de flores microbiennes anaérobies fermentant le glycérol en 1,3-propanediol.

A partir d'une flore microbienne prélevée dans des habitats anaérobies tels les digesteurs anaérobies d'effluents de distilleries, d'agro-industries , ainsi que des milieux anaérobies comme des sédiments et des boues de sites récepteurs d'effluents, l'obtention des souches pures bactériennes fermentant le glycérol est réalisée selon le protocole suivant.

Une fiole type pénicilline, contenant 120 ml de milieu minéral tamponné et du glycérol ( 20 g/l ) , est inoculée par 10 ml de la suspension bactérienne et incubée selon les conditions citées à l'exemple 3. En phase fermentaire du glycérol, contrôlée par analyse en HPLC, 90 ml de la suspension sont prélevés pour inoculer un réacteur anaérobie, contenant 0,9 1 de milieu de culture décrit ci-dessus . Durant la phase de production de propanediol observée par analyse HPLC, 1 ml de culture microbienne anaérobie du réacteur est prélevé en condition aseptique et anaérobie ( seringue purgée à l'azote ) pour inoculer un tube à bouchon à vis muni d'un septum, contenant 9 ml d'eau physiologique anaérobie stérile.

A partir du tube, des dilutions successives d'un facteur 10 sont réalisées de tube en tube . Pour chaque tube de dilution de 10³ à 10⁶ , deux boîtes de Pétri sont préparées: milieu brewer's ( Merck) en simple couche et milieu brewer's en double couche pour isolement des microorganismes anaérobies stricts. L'incubation se déroule en jarre anaérobie, purgée à l'azote , et maintenu sous anaérobiose par un dispositif " Anaerocult " de la Société Merck à 37°C. Après 48 h , les colonies sont prélevées et inoculées en tube de milieu glycérol (A) pour examen et identification après 24 h de culture selon les critères et méthodes de microbiologie préconisés pour les microorganismes anaérobies stricts et facultatifs.

Selon cette méthode trois souches :
- Enterobacter agglomerans, biogroupe V
- Clostridium butyricum
- Citrobacter amalonaticus
sont obtenues à partir des sources microbiennes suivantes : boues de digesteur anaérobie, boues et sédiments de milieux naturels ( sols, lagunes, étangs), à partir desquelles la production de 1,3-propanediol est observée.

Les deux souches : **Enterobacter agglomerans** et **Citrobacter amalonaticus,** appartiennent à des espèces bactériennes , non connues dans ia littérature scientifique pour la fermentation du glycérol . La nouvelle souche de C. butyricum obtenue est différente des souches connues de l'espèce par les profils fermentaires ( voir exemple 3).

### EXEMPLE 2:

### Production de 1,3-propanediol à partir de glycérol par Enterobacter agglomerans.

Le milieu nutritif utilisé pour la croissance et la production de 1,3-propanediol par **Enterobacter agglomerans** est un milieu minéral enrichi en extrait de levure et en sel de cobalt, contenant du glycérol comme seule source de carbone et d'énergie .

| Composition du milieu de culture | |
|---|---|
| KH₂PO₄ | 6 g |
| K₂HPO₄, 3H₂O | 10,7 g |
| (NH₄)₂SO₄ | 0,4 g |
| MgSO₄, 7H₂O | 0,2 g |
| CaCl₂ | 0,1 g |
| CoCl₂, 6H₂O | 1,8 mg |
| Extrait de levure | 1 g |
| Solution minérale | 1 ml |
| Glycérol | 20 g |
| H₂O distillée qsp | 1 l |

| Solution minérale | |
|---|---|
| EDTA | 5 g |
| H₃BO₃ | 0,124 g |
| MnCl₂, 4H₂O | 0,03 g |
| CoCl₂, 6H₂O | 0,02 g |
| FeSO₄, 7H₂O | 2 g |
| ZnSO₄, 7H₂O | 0,1 g |
| NaMoO₄, 2H₂O | 0,03 g |
| NiCl₂, 6H₂O | 0,02 g |
| Solution minérale (suite) | |
| CuCl₂, 2H₂O | 0,01 g |
| H₂O distillée qsp | 1 l |

Le milieu est porté à ébullition puis refroidi 5 sous azote . Après autoclavage, il est réduit par ajout d'une solution de L-cystéine à 20 gl⁻¹ ( 0,5 % vol/vol).

La pré-culture ( 90 ml) inoculée à partir de tubes de conservation ( 10 ml) est réalisée en flacons pénicilline anaérobies, à 37°C. Après 36 h d'incubation, l'inoculum activé est transféré dans un réacteur anaérobie de 1 l précédemment décrit, contenant 904,5 ml de milieu réduit à 2 % de glycérol et purgé à l'azote gazeux.

La fermentation se déroule à 37°C , à pH régulé à 7.

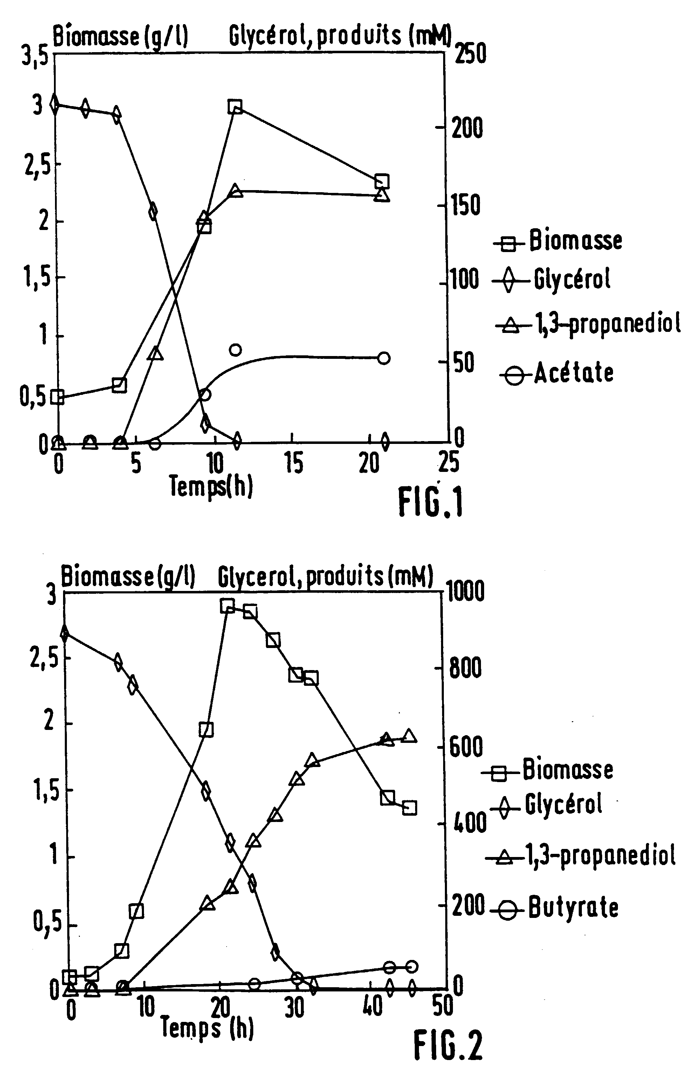

Le glycérol est exclusivement converti par Enterobacter agglomerans en 1,3-propanediol et acétate en tant que métabolites hydrocarbonés avec des rendements molaires respectifs de 74 mM de PPD et de 24 mM d'acétate formés à partir de 100 mM de glycérol consommé . Les résultats sont rassemblés à la figure 1 qui est un diagramme illustrant le profil de conversion du glycérol par Enterobacter agglomerans, dans lequel on a porté, en abscisses, la durée de conversion exprimée en heures et en ordonnées, respectivement sur l'axe de gauche la quantité de biomasse exprimée en g/l et sur l'axe de droite les quantités de glycérol et de produits formés (1,3-propanediol et acétate) , exprimées en mM.

La productivité volumique maximale en 1,3-propanediol est de 1,98 g de PPD/l/h, ce qui se traduit par une durée de fermentation de 12 heures.

### EXEMPLE 3:

### Production de 1,3-propanediol Dar Clostridium butyricum.

Clostridium butyricum est mis en culture pendant 24 heures sur milieu minéral fortement tamponné à base de glycérol dont la composition est décrite ci-dessous. L'incubation se déroule dans des flacons de type pénicilline, hermétiquement clos, de 120 ml , en conditions anaérobies strictes (atmosphère d'azote, ajout de composés réducteurs dans le milieu après autoclavage ) à 37°C . Le taux d'ensemencement est de 10% vol/vol.

Ces précultures sont ensuite utilisées pour inoculer des fermenteurs de 1 1 ( ajout de 90 ml de pré-culture dans 900 ml de milieu nutritif stérile ) maintenus en conditions anaérobies ( utilisation d'azote en phase gazeuse ) et munis de systèmes de régulation du pH (ajout contrôlé de soude 2N) et de température ( circulation d'eau thermostatée ) .

L'homogénéisation est assurée par agitation magnétique et un piège à oxygène à pyrogallol, placé à l'interface réacteur-atmosphère , permet d'éviter les entrées d'oxygène dans le réacteur .

Les fermentations ont lieu à 37°C , à pH contrôlé à 6,5 sur milieu salin contenant 10 % vol/vol de glycérol.

**TABLEAU I**

| Composition des milieux de culture | | |
|---|---|---|
| | (A) milieu tamponné (fiole) | (B) milieu pour réacteur pH régulé |
| K₂HPO₄ | 3,4 g | 1g |
| KH₂PO₄ | 1,3 g | 0,5 g |
| CaCO₃ | 2 g | 0 |
| (NH₄)₂SO₄ | 2 g | 2g |
| MgSO₄, 7H₂O | 0,2 g | 0,2g |
| CaCl₂, 2H₂O | 20 mg | 20mg |
| FeSO₄, 7H₂O | 5 mg | 5mg |
| Extrait de levure | 1 g | 1g |
| Glycérol | 20 g | 20 à 200 g |
| H₂O distillée | qsp 1000 ml | QSP 1000 ml |

Le milieu tamponné (A) est porté à ébullition, réparti dans des fioles hermétiquement bouchées (bouchon butyl et capsule métallique) et refroidi sous azote avant autoclavage.

Les réacteurs, après autoclavage, sont refroidis sous flux d'azote pour éviter la dissolution d'oxygène dans le milieu. Les solutions réductrices et vitaminiques ci-après sont ensuite ajoutées ( à 2% et 4% volume/volume respectivement ) dans ces milieux.

### Solution réductrice

6,25 g de L-cystéine et 6,25 g de Na₂S, 9H₂O sont dissous dans 500 ml d'une solution de soude 0,8 N préalablement désoxygénée.

### Solution vitaminique

| | |
|---|---|
| Biotine | 5 mg |
| Acide para-amino-benzoïque | 2 g |
| Cobalamide | 15 mg |
| H₂O distillée qsp | 100 ml |

Le profil de fermentation est présenté à la figure 2 qui est un diagramme analogue à la figure 1 , établi pour illustrer le profil de conversion du glycérol par Clostridium butyricum, les produits formés étant dans ce cas le 1,3-propanediol et le butyrate. Le rendement de la culture pure de Clostridium butyricum ( souche I-1211) est de 57 mM de 1,3-propanediol formé par 100 mM glycérol consommé. Le butyrate est le seul co-produit hydrocarboné détecté en phase aqueuse et représente moins de 7% de la quantité molaire de glycérol consommé .

Lorsque la fermentation débute avec 65 g glycérol/l, la productivité maximale de conversion en 1,3-propanediol est de 4,5 g/l/h et la durée de fermentation est de 22 heures.

### EXEMPLE 4:

### Production de 1,3-propanediol par Clostridium butyricum à partir de résidus industriels d'origine agricole et alimentaire contenant du glycérol.

Le microorganisme est pré-cultivé en conditions d'anaérobiose stricte, à 37°C en fioles pénicilline, sur milieu minéral fortement tamponné (A) décrit dans l'exemple 3, pendant 24 heures . Ces précultures sont ensuite utilisées pour inoculer un milieu d'origine industrielle dans lequel le glycérol est le composé organique majeur, représenté par des effluents de distillation d'alcool d'origine agricole.

Pour les fermentations présentées ci-après, les co-produits disponibles industriellement sont préparés selon le protocole suivant : ajustement de la teneur en glycérol entre 14 et 15,8 g/l par dilution du milieu et addition de facteurs nutritifs et salins (K₂HPO₄: 1 gl⁻¹; K₂HPO₄: 0,5 gl⁻¹; (NH₄)₂SO₄: 2 gl⁻¹; MgSO₄, 7H₂O: 0,2 gl⁻¹; CoCl₂,2H₂O: 20 mg⁻¹; FeSO₄, 7H₂O: 5 mgl⁻¹; 1 gl⁻¹ d'extrait de levure ).

Les réacteurs anaérobies contenant 0,9 l de milieu sont autoclavés et refroidis sous azote et réduits par addition de la solution réductrice citée à l'exemple 1.

L'inoculation est effectuée par 90 ml de suspension bactérienne , et la fermentation est conduite à pH 6,5 avec régulation et à 37°C.

**TABLEAU II**

| Caractéristiques de conversion de glycérol d'origine naturelle par Clostridium butyricum (souche I-1211) | | |
|---|---|---|
| Origine du substrat | Eau résiduaire de distillerie n°1 (vinasse de vin rouge) | Eau résiduaire de distillerie n° 2 (vinasse de vin blanc) |
| concentration initiale en glycérol | 15,8 g/l | 14g/l |
| production de 1,3-propanediol | 8,4 g/l | 6,6 g/l |
| rendement molaire mole de PPD produit/mole de glycérol consommé | 0,64 | 0,57 |

### EXEMPLE 5:

### Production de 1,3-propanediol par une population microbienne de digesteur anaérobie d'eaux résiduaires de distilleries de bio-éthanol, contenant les souches Clostridium butyricum ( souche I-1211) ou Enterobacter agglomerans (souche 1-1210).

La flore microbienne mixte anaérobie a été mise en culture selon le protocole de préparation des fermentations décrit dans l'exemple 3.

Le milieu de fermentation en réacteur anaérobie comprend du glycérol à concentration variable : entre 20 et 125 % ( poids/volume); les cultures se déroulent à pH compris entre 5 et 8, et à 37°C.

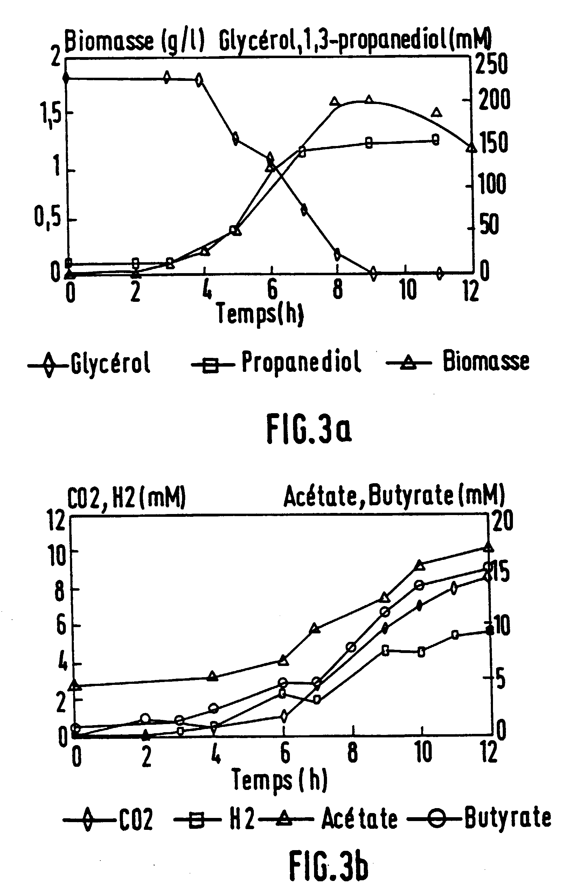

Les figures 3a et 3b montrent le profil de conversion du glycérol par la flore anaérobie de digesteur qui est une flore microbienne anaérobie mixte.

La figure 3a est un diagramme analogue aux figures 1 et 2 avec en ordonnées, à droite, les quantités en mM de glycérol et de 1,3-propanediol formés , tandis qu'à la figure 3b l'axe des ordonnées à gauche montre les quantités en mM de gaz (CO₂, H₂) formées et à droite , les quantités en mM de produits (1,3-propanediol et butyrate ) formés. Le 1,3-propanediol est le principal produit formé par fermentation du glycérol par la population microbienne anaérobie , avec un taux de conversion très élevé : 66,7 mM de 1,3-propanediol formé à partir de 100 mM de glycérol consommé . La vitesse maximale de production de 1,3-propanediol ( productivité volumique ) est de 2,3 g/l/hr, et pour une durée de fermentation de 11 heures . Le propanediol formé n'est pas consommé et s'accumule au cours de la fermentation . L'acétate et le butyrate sont formés dans des proportions molaires équivalentes pendant la phase de production de 1,3-propanediol, représentant respectivement 7,4 et 6,6 % (mM) du glycérol consommé . Le CO₂ et l'hydrogène sont les produits gazeux formés .

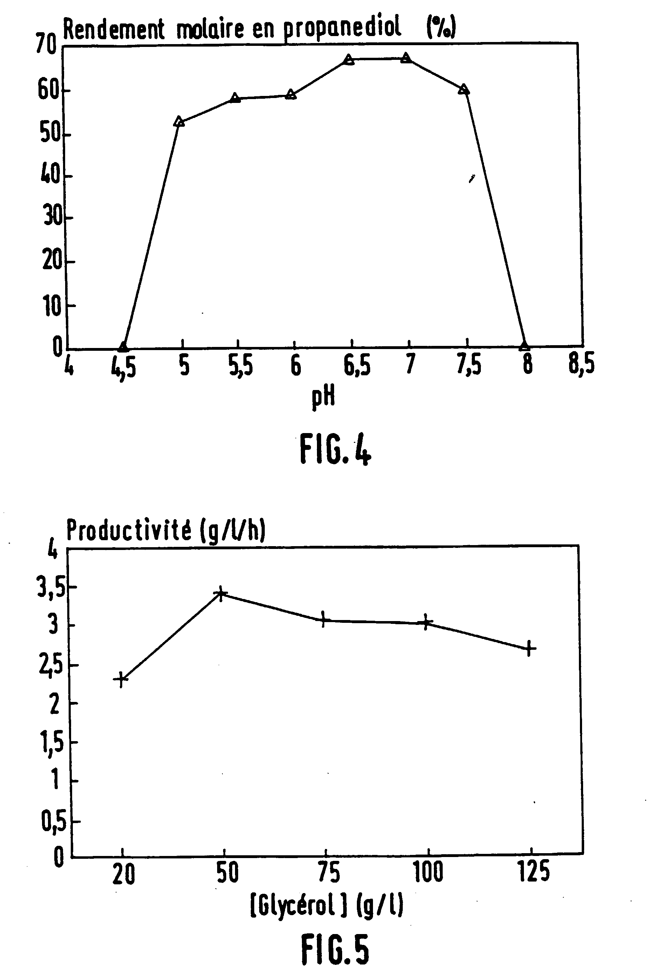

Les effets des conditions de pH et de la teneur en glycérol ont été illustrés aux figures 4 et 5, respectivement.

La figure 4 montre l'effet du pH sur le rendement molaire de production de 1,3-propanediol par la flore microbienne anaérobie mixte définie à l'exemple 5.

Il s'agit d'un diagramme dans lequel on a porté le pH en abscisses et , en ordonnées, le rendement molaire en 1,3-propanediol, exprimé en %.

La figure 5 montre l'effet de la concentration en glycérol sur la vitesse maximale de production de 1,3-propanediol pour la même flore microbienne anaérobie mixte . Il s'agit d'un diagramme dans lequel on a porté en abscisses la quantité de glycérol exprimée en g/l et en ordonnées la productivité, exprimée en g/l/h, de 1,3-propanediol formé.

D'un point de vue rendement de conversion du glycérol en 1,3-propanediol, le pH le plus favorable est compris entre 6,5 et 7; rendement de 66,7 % ( mM propanediol formé pour 100 mM de glycérol consommé ) . Mais la production de propanediol s'effectue à pH 7,5 et jusqu'à pH 5, où le rendement est encore supérieur à 50%. Au niveau cinétique, la plus forte productivité est obtenue à pH 6,5 et pour une concentration initiale en glycérol de 50 g/l : 2,4 g 1,3-propanediol formé par litre et par heure.

## Revendications

1. Procédé pour l'obtention de produits à activité bactérienne , capables de transformer le glycérol en 1,3-propanediol , ledit procédé comprenant les étapes de :
a) préculture de populations anaérobies , issues d'habitats microbiens anaérobies , ladite préculture étant réalisée dans des conditions anaérobies sur milieu nutritif tamponné contenant du glycérol comme seule source de carbone,
b) isolement des précultures microbiennes actives capables de fermenter le glycérol;
c) enrichissement par fermentation discontinue desdites précultures dans un réacteur anaérobie , sur milieu nutritif à base de glycérol comme substrat et à pH régulé ;
d) isolement des produits à activité bactérienne capables de transformer le glycérol en 1,3-propanediol.

2. Procédé selon la revendication 1, caractérisé en ce qu'à titre d'habitats microbiens anaérobies , on utilise des digesteurs anaérobies , par exemple d'effluents de distillerie, d'agro-industrie ou bien des milieux anaérobies tels que des sédiments et des boues de sites récepteurs d'effluents.

3. Procédé selon l'une des revendications 1 ou 2, caractérisé en ce qu'on règle le pH à une valeur comprise entre 5 et 8, et de préférence entre 6 et 7,5.

4. Procédé selon l'une quelconque des revendications 1 à 3, caractérisé en ce que l'isolement des souches constitutives des produits à activité bactérienne est opéré à partir de prélévements en anaérobiose, avec dilution des populations puis étalement sur boîtes de Pétri sur milieux de cultures gélosés , dans des conditions anaérobies .

5. Produits à activité bactérienne, capables de transformer ie glycérol en 1,3-propanediol , susceptibles d'être obtenus par le procédé selon l'une quelconque des revendications 1 à 4.

6. Produits selon la revendication 5, comprenant des bactéries Enterobacter agglomerans, Clostridium butyricum cu Citrobacter amalonaticus, sous forme de cultures mixtes d'écosystèmes anaérobies.

7. Produits selon l'une des revendications 5 ou 6 consistant en des bactéries appartenant à l'espèce Enterobacter agglomerans .

8. Souche Enterobacter agglomerans , inscrite à la CNCM sous le n° I-1210 .

9. Souche de Clostridium butyricum inscrite à la CNCM sous le n° I-1211 .

10. Souche de Citrobacter amalonaticus ,inscrite à la CNCM sous le n°I-1212.

11. Application des produits , espèces et souches à activité bactérienne selon l'une quelconque des revendications 5 à 10, en vue de la conversion du glycérol par fermentation en 1,3-propanediol .

12. Application selon la revendication 11, selon laquelle on utilise l'espèce Enterobacter agglomerans , en particulier la souche Enterobacter agglomerans I-1210, la conversion du glycérol en 1,3-propanediol étant réalisée en condition d'anaérobiose avec contrôle et régulation du pH entre 6 et 7,5 et de préférence à pH 7, dans une gamme de température située entre 28 et 40°C environ .

13. Application selon la revendication 11, dans laquelle on utilise la souche Clostridium butyricum I-1211 , la conversion du glycérol en 1,3-propanediol étant réalisée en anaérobiose , avec pH régulé entre 5 et 8, et de préférence entre 6 et 7,5 , et à une température située entre 32 et 40°C.

14. Application selon la revendication 11 , dans laquelle on utilise une population bactérienne anaérobie mixte , en particulier une population bactérienne, contenant les espèces Enterobacter agglomerans et Clostridium butyricum et/ou les souches Clostridium butyricum I-1211 et Enterobacter agglomerans I-1210.

15. Appli cation de produits à activité bactérienne selon la revendication 5, consistant en des populations bactériennes mixtes fermentant le glycérol et produisant du 1,3-propanediol, lesdites populations contenant notamment la souche Citrobacter amalonaticus I-1212 selon la revendication 10.

## Patentansprüche

1. Verfahren zur Gewinnung von Produkten mit bakterieller Aktivität, die in der Lage sind, Glycerin in 1,3-Propandiol umzuwandeln, wobei das Verfahren die Schritte umfaßt:
a) Vorkultur anaerober Populationen, die aus anaeroben Mikrobenhabitaten stammen, wobei die Vorkultur unter anaeroben Bedingungen auf einem gepufferten Nährmedium, das Glycerin als einzige Kohlenstoffquelle enthält, durchgeführt wird,
b) Isolierung aktiver mikrobieller Vorkulturen, die Glycerin vergären können,
c) Anreicherung der Vorkulturen durch diskontinuierliche Fermentation in einem anaeroben Reaktor auf einem Nährmedium auf der Basis von Glycerin als Substrat mit eingestelltem pH-Wert,
d) Isolierung von Produkten mit bakterieller Aktivität, die in der Lage sind, Glycerin in 1,3-Propandiol umzuwandeln.

2. Verfahren gemäß Anspruch 1, dadurch gekennzeichnet, daß als anaerobe Mikrobenhabitate anaerobe Digestoren, zum Beispiel von Destillerie- oder Agroindustrieabwässern, oder anaerobe Medien, wie Sedimente und Schlämme aus Abwasseraufnahmegebieten, verwendet werden.

3. Verfahren gemäß einem der Ansprüche 1 oder 2, dadurch gekennzeichnet, daß der pH auf einen Wert zwischen 5 und 8 und vorzugsweise zwischen 6 und 7,5 eingestellt wird.

4. Verfahren gemäß einem der Ansprüche 1 bis 3, dadurch gekennzeichnet, daß die Isolierung der Stämme, die den Produkten mit bakterieller Aktivität zugrundeliegen, ausgehend von in Anaerobiose befindlichen Proben unter Verdünnen der Populationen und Ausstreichen auf Petri-Schalen auf Gelose-Kulturmedien unter anaeroben Bedingungen erfolgt.

5. Produkte mit bakterieller Aktivität, die in der Lage sind, Glycerin in 1,3-Propandiol umzuwandeln, und die nach dem Verfahren gemäß einem der Ansprüche 1 bis 4 erhalten werden können.

6. Produkte gemäß Anspruch 5, die die Bakterien *Enterobacter agglomerans, clostridium butyricum* oder *citrobacter amalonaticus* in Form von Mischkulturen anaerober Ökosysteme umfassen.

7. Produkte gemäß einem der Ansprüche 5 oder 6, die aus Bakterien bestehen, die zur Spezies *Enterobacter agglomerans* gehören.

8. Stamm von *Enterobacter agglomerans,* der bei der CNCM unter der Nr. I-1210 hinterlegt ist.

9. Stamm von *Clostridium butyricum,* der bei der CNCM unter der Nr. I-1211 hinterlegt ist.

10. Stamm von *Citrobacter amalonaticus,* der bei der CNCM unter der Nr. I-1212 hinterlegt ist.

11. Verwendung der Produkte, Spezies und Stämme mit bakterieller Aktivität gemäß einem der Ansprüche 5 bis 10 zur Umwandlung von Glycerin in 1,3-Propandiol durch Gärung.

12. Verwendung gemäß Anspruch 11, wobei die Spezies *Enterobacter agglomerans,* insbesondere der Stamm *Enterobacter agglomerans* I-1210, verwendet wird, wobei die Umwandlung von Glycerin in 1,3-Propandiol unter den Bedingungen einer Anaerobiose unter Kontrolle und Einstellung des pH-Werts zwischen 6 und 7,5 und vorzugsweise auf pH 7 in einem Temperaturbereich zwischen ungefähr 28 und 40°C durchgeführt wird.

13. Verwendung gemäß Anspruch 11, wobei der Stamm *Clostridium butyricum* I-1211 verwendet wird, wobei die Umwandlung von Glycerin in 1,3-Propandiol in Anaerobiose mit zwischen 5 und 8 und vorzugsweise zwischen 6 und 7,5 eingestelltem pH-wert bei einer Temperatur zwischen 32 und 40°C durchgeführt wird.

14. Verwendung gemäß Anspruch 11, wobei eine gemischte anaerobe Bakterienpopulation, insbesondere eine Bakterienpopulation, die die Spezies *Enterobacter agglomerans* und *Clostridium butyricum* und/oder die Stämme *Clostridium butyricum* I-1211 und *Enterobacter agglomerans* I-1210 enthält, verwendet wird.

15. Verwendung von Produkten mit bakterieller Aktivität gemäß Anspruch 5, die aus gemischten Bakterienpopulationen bestehen, die Glycerin vergären und 1,3-Propandiol erzeugen, wobei die Populationen insbesondere den Stamm *Citrobacter amalonaticus* I-1212 gemäß Anspruch 10 enthalten.

## Claims

1. Process for the production of products having bacterial activity and capable of converting glycerol into 1,3-propanediol, said process comprising the steps of
(a) preculturing anaerobic populations, derived from anaerobic microbial habitats, said preculture being carried out under anaerobic conditions on a buffered nutrient medium containing glycerol as the sole carbon source,
(b) isolating the active microbial precultures capable of fermenting glycerol,
(c) enriching said precultures by discontinuous fermentation in an anaerobic reactor, on a nutrient medium based on glycerol as substrate and at controlled pH,
(d) isolating the products having bacterial activity and capable of converting glycerol into 1,3-propanediol.

2. Process according to claim 1, characterized in that by way of anaerobic microbial habitats can be used anaerobic digesters, for example for effluents from distilleries, agroindustries, or anaerobic media such as sediments and muds from effluent receiving sites.

3. Process according to one of claims 1 or 2, characterized in that the pH is controlled to between 5 and 8, and preferably between 6 and 7.5.

4. Process according to any one of claims 1 to 3, characterized in that the isolation of the strains constituent of the products having a bacterial activity is carried out by removing samples under anaerobic conditions, with dilution of the populations, then spreading onto Petri dishes on agar culture media, under anaerobic conditions.

5. Products having bacterial activity and capable of converting glycerol into 1,3-propanediol, able to be produced by the process according to any one of claims 1 to 4.

6. Products according to claim 5, comprising the bacteria Enterobacter agglomerans, Clostridium butyricum, or Citrobacter amalonaticus, in the form of mixed cultures of anaerobic ecosystems.

7. Products according to claims 5 or 6 consisting of bacteria belonging to the species Enterobacter agglomerans.

8. Enterobacter agglomerans strain, registered at the CNCM under the N°I-1210.

9. Clostridium butyricum strain, registered at the CNCM under the N°I-1211.

10. Citrobacter amalonaticus strain, registered at the CNCM under the N°I-1212.

11. Application of the products, species and strains having bacterial activity according to any one of claims 5 to 10, to the conversion of glycerol into 1,3-propanediol by fermentation.

12. Application according to claim 11, according to which the species Enterobacter agglomerans, and in particular the strain I-1210 of Enterobacter agglomerans, is used, the conversion of glycerol into 1,3-propanediol being carried out under anaerobic conditions with control and regulation of the pH to between 6 and 7.5 and preferably at pH 7, in a temperature range of between approximately 28 and 40°C.

13. Application according to claim 11, in which Clostridium butyricum strain I-1211 is used, the conversion of glycerol into 1,3-propanediol being carried out under anaerobic conditions, with control of the pH to between 5 and 8, and preferably between 6 and 7.5, and at a temperature between 32 and 40°C.

14. Application according to claim 11, in which a mixed anaerobic bacterial population is used, in particular a bacterial population containing the species Enterobacter agglomerans and Clostridium butyricum and/or the strains Clostridium butyricum I-1211 and Enterobacter agglomerans I-1210.

15. Application of products having bacterial activity according to claim 5, comprising mixed bacterial populations which ferment glycerol and produce 1,3-propanediol, said populations in particular containing Citrobacter amalonaticus strain 1212 according to claim 10.
